# EUROPEAN PATENT SPECIFICATION

(11) **EP 1 609 462 B1**
(45) Date of publication and mention of the grant of the patent: **29.07.2015**
(21) Application number: 05101620.2
(22) Date of filing: 03.03.2005
(51) Int. Cl.: A61K 8/44, A61K 8/65, A61K 8/73, A61K 31/722, A61K 31/726, A61K 31/737, A61L 15/12, A61L 27/26, A61Q 19/00

(54) **Cosmetic or dermatological preparation comprising a nutrient medium phase**
Kosmetische oder dermatologische Zubereitung umfassend eine Nährmedienphase
Préparation cosmétique ou dermatologique comprenant un milieu nutritif

(30) Priority: 22.04.2004 DE 102004020035
(43) Date of publication of application: 28.12.2005
(73) Proprietor: La Prairie Group AG, 8604 Volketswil / Zürich (CH)
(72) Inventor: Mönks, Monica, 3185 Schmitten (CH); Evangelisti, Carmen, 8427 Freienstein (CH); Ibanez, Sibylle, 8707 Uetikon am See ZH (CH); Gohla, Sven Dr., 21077 Hamburg (DE)
(74) Representative: Hartmann, Jost

(56) References cited:
- WO-A-98/16629
- WO-A-98/22114
- US-A- 4 414 202
- US-A- 5 489 304
- US-A- 5 759 570
- US-A- 6 043 092
- US-A- 6 057 148
- SHAHABEDDIN L ET AL: "CHARACTERIZATION OF SKIN RECONSTRUCTED ON A CHITOSAN-CROSS-LINKED COLLAGEN-GLYCOSAMINOGLYCAN MATRIX" SKIN PHARMACOLOGY, S. KARGER, BASEL, CH, vol. 3, no. 2, 1990, pages 107-114, XP000673928 ISSN: 1011-0283
- PATENT ABSTRACTS OF JAPAN vol. 2003, no. 07, 3 July 2003 (2003-07-03) & JP 2003 062057 A (NEXT:KK), 4 March 2003 (2003-03-04)

## Description

### BACKGROUND OF THE INVENTION

The present invention relates to a cosmetic method, wherein the cosmetic preparation is obtainable by combining collagen, chitosan and glycosylaminoglycan with one or more skin cell culture media and substances which exhibit a beneficial effect when applied to healthy skin. The preparation comprises at least one nutrient medium phase for skin cells or corneal cells comprising the above collagen/chitosan/glycosylaminoglycan combination.

Various circulations exist within the human body, such as the blood circulation, the lymphatic system and the intracellular and extracellular tissue fluids. The composition of the solvent "water" with its mineral and bioorganic constituents in these various "transport media" is approximately the same and is based, highly simplified, on salts, amino acids, vitamins, sugars, proteins and proteids, and trace elements. During evolution, our body has learnt to create within these fluids "communication networks" and nutritional strategies, and an equilibrium of catabolic to anabolic processes, which make the complex life of our multicellular body possible. In this environment, our body has learnt to construct from its "single individuals", the cells, a complicated but efficient network of direct and mediator-related contacts. These "communication pathways" function efficiently and harmlessly only if the natural dynamic equilibrium of our body, the so-called "homeostasis", is maintained. If cells are removed from the tissue assemblage or if the homeostasis in the tissue assemblage is impaired, it is no longer possible for individual cells to exist or for tissues to function healthily. The medical and biosciences have for decades looked for possibilities of cultivating tissues or individual cells in suitable environmental conditions outside the body. This was successful only when it was possible to simulate as perfectly as possible the living conditions in the body for the single cells or tissue constituents to be cultivated.

Thus, if cells are removed from intact tissue, they must be cultivated in environments which come as close as possible to the natural living conditions in the body. Requirements for this are supply and transport away of nutrients, and the presence of vital factors.

These environments are well-defined compositions of mineral and biomaterials which are known in science as cell culture media. Cell culture media are obtainable from suitable specialist retailers as powder or liquid media and have slightly different compositions depending on the nature of the cells or tissue constituents to be cultivated. Cell culture media are used in liquid form. With a suitable composition, they make it possible to maintain or even multiply microorganisms or cells in culture, i.e., outside the body.

In the course of tissue research it has been possible to identify and investigate the individual needs of cells and cells in intact tissues. In this connection, the ratio of mineral and bioorganic substances of a cell culture medium is slightly variable from cell type to cell type and must be ascertained accurately for optimized survival and growth. The composition of the cell culture medium always depends on the requirements of the cells to be grown. A distinction is made between synthetic media, whose ingredients are accurately known on the basis of pure substances, and complex media, whose exact composition may vary and is in part not accurately known. Cell culture media usually comprise, besides water, a carbon source and a nitrogen source, phosphate compounds and sulfur compounds, and minerals and, optionally, growth promoters and/or vitamins.

If the compositions of the media are suitable, the cells are able to multiply and produce the factors necessary for survival *"in situ"* by themselves.

In order to generate good growth of the cells, serum is frequently added to the cell culture media. The serum has a complicated composition and provides the cells with, *inter alia*, hormones, adhesion factors, and amino acids. Culture media which contain serum are, however, costly and do not allow thermal sterilization. One therefore usually tries to make do with media which contain no serum. Serum-free culture media make it possible to cultivate cells under controlled and defined conditions, so that unwanted effects due to variations in the serum composition are eliminated. In addition, contamination of the cell cultures with viruses and bacteria is reduced when using serum-free media.

It is known that skin cells can be kept alive particularly well-preserved and for long periods of time and can even be induced to grow and differentiate in one-, two- and three-dimensional cultures by optimizing the ingredients in the culture medium. It has also been possible to demonstrate that suitable media also make possible the production of growth factors *in situ*.

When there are extreme changes in the skin resulting from extensive burns or chills, the integrity and the functionality of the cutaneous tissue may be so impaired that the skin is no longer able to regenerate on its own. The body responds to such severe events with hyperthermia, massive release of mediators of inflammation and irritation, and with an enormous loss of fluid, which in the past has always and inevitably resulted in the death of people with severe burns. Burns and chills which have led to losses of cutaneous tissue can be compensated by skin transplants and thus the skin can be closed. However, this is successful only if sufficient remaining skin is available for transplantation. In cases of burns of more than 60% of the total cutaneous tissue, transplantation on its own is usually of no assistance. It is necessary to re-produce viable tissue from the remaining skin cells. In this connection, because of the rejection reactions between non-HLA-compatible tissues, it is not possible to take allogeneic skin or allogeneic skin cells. It is therefore necessary to form a new cutaneous tissue *in situ* from the remaining viable skin cells.

The hornified epidermis forms the protective shield of the skin. For this function to be optimally exercised it is necessary for the skin cells (keratinocytes) to pass through the process of so-called epidermal differentiation. After division of the cells in the basal layer, the keratinocytes migrate to the skin surface and undergo a number of changes during this, until they form the horny layer (stratum corneum) as dead, flat, anuclear corneocytes, and eventually are desquamated. During the epidermal differentiation there is formation of various proteins having specific functions. These include, *inter alia*, keratins, involucrin, filaggrin and transglutaminase. For optimal formation of the epidermis and the horny layer it is necessary for these proteins to be formed in coordinated fashion and in sufficient quantity.

Many cosmetics, skin care products or wound healing products which help to compensate or at least reduce the disorders of the skin are known in the prior art.

Thus, for example, geroderma is cosmetically treated primarily with vitamin A derivatives or hydroxy acids which lead, via stimulation of the proliferation of the basal cells in the epidermis, to a thickening of the epidermis and thus smoothing of the skin. More recent approaches consist of targeted replacement of the proteins which are absent or present in reduced quantity in dry skin or geroderma, or indirect intervention in the metabolic processes which are disturbed in dry skin or with increasing age, in order to normalize them. An example which may be mentioned here is stimulation of collagen synthesis with the aim of reducing wrinkles. In addition, for example, laminin, substances for prolonging the lifetime of skin cells and certain extracts for stimulating epidermal differentiation are employed. However, some of these are pharmacologically active substances with a high potential for side effects.

US 6057148 dislose preparations which comprise collagen, but which does not comprise skin cell culture media. The cultured fibroplasts and the medium were removed.

US 5489304 disclose a method for regenerating skin at a burn or wound site comprising the steps of applying a bilayer membrane, which comprises Collagen, Glycosylaminoglycan and a cell culture media wich comprises necessarily genetically unmodified cells.

None of the preparations known from the prior art on their own enable the skin to reconstitute/regenerate itself without displaying unwanted side effects.

It would be advantageous to have available a preparation which enables the skin to regenerate itself without displaying unwanted side effects.

EP 296078, EP 462426 and U.S. Patent Nos. 5,116,824, 6,541,023 and 5,808,050, disclose preparations which comprise chitosans, collagens and glycosylaminoglycans. However, none of these documents discloses preparations which support the regeneration of the skin, lastingly improve the skin structure and help the skin to regain its elasticity and healthy appearance.

The present invention provides a cosmetic skin care method, wherein the method comprises applying to a healthy skin a cosmetic preparation which is obtainable by combining certain substances. These substances comprise
(a) collagen and/or a derivative thereof;
(b) chitosan and/or acetylated chitosan with a degree of acetylation of up to about 50 %;
(c) at least one glycosylaminoglycan ;
(d) at last one substance selected from amino acids (including the HCl salts thereof, preferably L-amino acids and even more preferred, essential amino acids), α-biotin, (NH₄)₆Mo₇O₂₄, adenine, AlCl₃, biotin, CaCl₂, calcium pantothenate, choline chloride, CoCl₂, CrK(SO₄)₂, CuSO₄, D-Ca pantothenate, EDTA.Na₂, EDTA.Na₃, Fe(NO₃)₃, FeSO₄, folic acid, glucose, H₂SeO₃, HEPES, hypoxanthine, insulin human, KCI, linoleic acid, lipoic acid, MgCl₂, MnCl₂, MnSO₄, myo-inositol, Na₂HPO₄, Na₂SeO₃, Na₂SiO₃, NaCl, NaH₂PO₄, NaHCO₃, sodium pyruvate, sodium acetate, NH₄VO₃, NiCl₂, nicotinamide, phenol red, polysorbate 80, putrescine, putrescine 2HCl, pyridoxine HCl, pyridoxal HCl, riboflavin, SnCl₂, thiamine HCl, thymidine, vitamin B₁₂, and ZnSO₄ and
(e) one or more skin cell culture media.

In one aspect of this preparation, the amino acids may comprise one or more substances selected from L-alanine, L-arginine, L-asparagine, L-aspartic acid, L-cysteine, L-cystine, glycine, L-glutamine, L-glutamic acid, L-histidine, L-isoleucine, L-leucine, L-lysine, L-methionine, L-phenylalanine, L-proline, L-serine, L-threonine, L-tryptophan, L-tyrosine, L-valine and the hydrochloride salts thereof.

In another aspect, at least two substances from the above group (d) may be employed, e.g., at least five, at least ten, at least twenty, at least fifty substances, or all substances from group (d).

In yet another aspect of the preparation of the present invention, the substances from group (d) may comprise at least one substance, e.g., at least two, three, four, five, six, seven or all of the substances selected from the group of glucose, folic acid, L-lysine, L-threonine, L-arginine, L-serine, L-histidine and glycine. In this regard, whenever in the present specification and the appended claims reference is made to an amino acid, this reference also includes the hydrochlorids salt of the amino acid. For example, "L-lysine" is intended to mean "L-lysine and/or L-lysine HCl".

In a still further aspect, at least one of the substances (a) and (b) may be of marine origin or of synthetic origin.

In another aspect, the collagen (a) may comprise one or more collagens selected from types 1, 3, 4 and 5 and/or the chitosan (b) may comprise chitosan having a molecular weight of from about 80,000 g/mol to about 15,000,000 g/mol and/or chitosan obtained from crustaceans and/or insects and/or the glycosylaminoglycan (c) may comprise chondroitin 4-sulfate and/or chondroitin 6-sulfate.

In another aspect, the substances (a) to (c) may be employed in a total amount of from about 0.0005 % to about 50 % by weight, based on the total weight of the preparation, e.g., in a total amount of from about 0.0015 % to about 30 % by weight, from about 0.005 % to about 10 % by weight, from about 0.01 % to about 1 % by weight, or from about 0.015 % to about 0.1 % by weight.

In another aspect of the preparation of the present invention, the weight ratio of the substances (a) and (c) may be from about 35 : 1 to about 3 : 1, e.g., from about 20 : 1 to about 6 : 1, or from about 10 : 1 to about 8 : 1.

In yet another aspect of the preparation, the weight ratio of the substances (a) and (b) may be from about 10 : 1 to about 1.5 : 1, e.g., from about 7 : 1 to about 2.5 : 1, or from about 5 : 1 to about 3.5 : 1.

In a still further aspect, the weight ratio of the substances (b) and (c) may be from about 10 : 1 to about 1 : 1, e.g., from about 5 : 1 to about 1.5 : 1, or from about 3 : 1 to about 2 : 1.

For example, the skin cell culture media may comprise DMEM/HAM F12 (1:1) and/or MCDB 153.

In yet another aspect, the preparation may comprise a nanosponge matrix and/or a microsponge matrix formed by the substances (a) to (c), which matrix has been reconstituted in cell culture media. By way of non-limiting example, the cell culture media may be selected from physiological saline solution, nutrient media and complete media for culturing primary body cells, e.g., culture media for primary fibroblasts and keratinocytes. The complete media may, for example, be supplemented with serum substitutes.

In another aspect, the preparation may further comprise one or more of a citrate buffer, Q10, alpha-glucosyl rutin, Zn orotate, carnitine, creatine and taurine and/or one or more alpha-hydroxy acids.

In another aspect, the preparation may further comprise water, for example, at least about 30 % by weight of water, based on the total weight of the preparation, e.g., at least about 40 %, at least about 50 %, at least about 60 %, at least about 70 %, at least about 80 %, at least about 90 %, or at least about 95 % by weight of water.

The preparation might be in a form which is selected from an aqueous gel, an O/W emulsion, a W/O/W emulsion, a W/O emulsion, a microemulsion and a cosmetic stick.

The preparation might further be in a form which is selected from an aqueous surfactant preparation, an emulsion, an ointment, a cream, a gel, a dusting powder, a mask, a matrix bandage, a gel bandage, a foam preparation and an aerosol preparation.

The preparation might also be in the form of an article which is selected from a skin covering, a patch, a pad, a tissue and a bandage.

In one aspect, the preparation may comprise all of glucose, folic acid, L-lysine, L-threonine, L-arginine, L-serine, L-histidine and glycine.

In another aspect, the substances (a) to (c) may be employed in a total amount of from about 0.015 % to about 0.1 % by weight and/or the weight ratio of substances (a) and (c) may be from about 10 : 1 to about 8 : 1 and/or the weight ratio of substances (a) and (b) may be from about 5 : 1 to about 3.5 : 1 and/or the weight ratio of substances (b) and (c) may be from about 3 : 1 to about 2 : 1.

In yet another aspect of the preparation, one or more substances selected from α-biotin, (NH₄)₆Mo₇O₂₄, adenine, AlCl₃, biotin, CaCl₂, calcium pantothenate, choline chloride, CoCl₂, CrK(SO₄)₂, CuSO₄, D-Ca pantothenate, EDTA.Na₂, EDTA.Na₃, Fe(NO₃)₃, FeSO₄, H₂SeO₃, HEPES, hypoxanthine, insulin human, KCI, linoleic acid, lipoic acid, MgCl₂, MnCl₂, MnSO₄, myo-inositol, Na₂HPO₄, Na₂SeO₃, Na₂SiO₃, NaCl, NaH₂PO₄, NaHCO₃, sodium pyruvate, sodium acetate, NH₄VO₃, NiCl₂, nicotinamide, phenol red, polysorbate 80, putrescine, putrescine 2HCl, pyridoxine HCl, pyridoxal HCl, riboflavin, SnCl₂, thiamine HCl, thymidine, vitamin B₁₂, ZnSO₄, L-alanine, L-asparagine, L-aspartic acid, L-cysteine, L-cystine, glycine, L-glutamine, L-glutamic acid, L-histidine, L-isoleucine, L-leucine, L-methionine, L-phenylalanine, L-proline, L-tyrosine and L-valine may additionally be employed therein.

In a still further aspect of the preparation, the collagen may comprise one or more collagens selected from types 1, 3, 4 and 5 and/or the chitosan may comprise chitosan having a molecular weight of from about 80,000 g/mol to about 15,000,000 g/mol and/or the glycosylaminoglycan may comprise chondroitin 4-sulfate and/or chondroitin 6-sulfate.

The present invention provides a skin care method which comprises applying to the skin the preparation of the present invention, including the various aspects thereof.

Regarding the weight ratios of the components (a) to (d) of the preparation of the present invention, it is noted that the weight ratio (a) : (b) will usually be not higher than about 10 : 1, e.g., not higher than about 8 : 1, not higher than about 7 : 1, not higher than about 6 : 1, or not higher than about 5 :1, and will usually be not lower than about 1.5 : 1, e.g., not lower than about 2 : 1, not lower than about 2.5 : 1, not lower than about 3 : 1, or not lower than about 3.5 : 1. The weight ratio (a) : (c) will usually be not higher than about 35 : 1, e.g., not higher than about 30 : 1, not higher than about 25 : 1, not higher than about 20 : 1, not higher than about 15 : 1, or not higher than about 10 :1, and will usually be not lower than about 4 : 1, e.g., not lower than about 5 : 1, not lower than about 6 : 1, not lower than about 7 : 1, or not lower than about 8 : 1. The weight ratio (b) : (c) will usually be not higher than about 10 : 1, e.g., not higher than about 8 : 1, not higher than about 6 : 1, not higher than about 5 : 1, not higher than about 4 : 1, or not higher than about 3 :1, and will usually be not lower than about 1 : 1, e.g., not lower than about 1.5 : 1, or not lower than about 2 : 1.

Regarding the weight ratio of the total substances [(a) + (b) + (c)] and the substances of group (d), the ratio [(a) + (b) + (c)] : (d) will usually be from about 100 : 1 to about 1 : 100. When one or more substances from the group glucose, folic acid, L-lysine, L-threonine, L-arginine, L-serine, L-histidine and glycine are employed, the weight ratio of [(a) + (b) + (c)] and the total amount of these substances (d) will usually be not higher than about 30 : 1, e.g., not higher than about 20 : 1, or not higher than about 10 : 1, and not lower than about 1 : 20, e.g., not lower than about 1 : 15, or not lower than about 1 : 10.

In addition to the use of glucose, folic acid, L-lysine, L-threonine, L-arginine, L-serine, L-histidine and/or glycine, the use of L-tryptophan and/or calcium pantothenate for the manufacture of the preparation of the present invention may also be of particular advantage.

The substances (a) to (c) will usually be employed in a total amount of from about 0.00001 % by weight to about 99 % by weight, based on the total weight of the preparation. However, frequently the total amount will be not higher than about 50 % by weight, e.g., not higher than about 30 % by weight, not higher than about 10 % by weight, not higher than about 1 % by weight, or not higher than about 0.1 % by weight, but not lower than about 0.0001 % by weight, e.g., not lower than about 0.0015 % by weight, not lower than about 0.005 % by weight, not lower than about 0.01 % by weight, or not lower than about 0.015 % by weight.

It has unexpectedly been found that a cosmetic preparation which comprises a mixture that is obtainable by combining collagen , chitosan (and/or acetylated chitosan with a degree of acetylation not exceeding about 50 %) and a glycosylaminoglycan with one or more (e.g., all) of the substances from group (d) is particularly effective in enabling the skin to reconstitute itself without displaying unwanted side effects to any substantial extent.

Preparations according to the present invention comprise one or more skin cell culture media, preferably at least DMEM/HAM F12 (1:1) and/or MCDB 153, are particularly advantageous. In addition, all culture media which permit the culture of healthy differentiated skin (3-D models) and, in particular, media which may be used to culture primary fibroblasts and/or keratinocytes and make complete reconstitution of the skin possible are contemplated as culture media for use in the present invention. Serum substitutes for serum-free cell cultures may also advantageously be used, although they are not indispensible.

A particularly advantageous combination according to the present invention comprises cell-nourishing culture media, preferably media for cultivating skin cultures or corneal cultures of all types, with a cellular matrix that comprises collagens, acetylated chitosans with a degree of acetylation of up to about 50%, preferably up to about 40%, and chondroitin sulfates. This combination by itself, or mixed with a cosmetic preparation or incorporated into a natural or synthetic polymer matrix such as, e.g., a polyurethane matrix is extremely efficient with respect to skin regeneration/reconstitution, skin care.

A process for the preparation of a matrix that may be used in the present invention is described in, e.g., EP 296078, mentioned above.

It has unexpectedly been found that it is possible to obtain the matrix described in EP 296078 by using entirely marine and/or synthetic raw material sources and that the results are the same as those obtainable with the matrix of EP 296078.

In one aspect, the preparation of the present invention may be described as comprising a primary microporous or nanoporous matrix which preferably comprises marine collagens selected from the group of type 3, type 1, type 4 and/or type 5 or blends thereof, chitosans, preferably with a molecular weight of from about 80,000 D to about 15,000,000 D and with a degree of acetylation of from about 5 % to about 50 %, blended with a mixture of chondroitin 4-and 6-sulfates, which are preferably employed in an amount of from about 3 % to about 15 % by weight based on the amount of the employed collagens. The matrix can be imagined to be in the form of a microtubular or nanotubular sponge. On lyophilization, the composition of the described molecules generates a nano- or microsponge (matrix).

The matrix may be composed of an aerogel prepared by lyophilization, which matrix may be introduced into the aqueous phases, active ingredient phases and/or culture media phases of finished cosmetic preparations. In this case, the aerogel may be converted into a hydrogel, or may be processed as aerogel, for example, together with (and/or into) a polyurethane matrix or a silicone matrix.

It has been shown that the preparation according to the present invention, which comprises cell culture media, results in advantages in the morphology and growth rate of primary human keratinocytes and fibroblasts from young and old donors *in vitro.* In principle, all growth and maintenance media are suitable for this purpose, but those which are adapted to the requirements of skin cells and enable the construction of "new skin" from individual dermis and/or epidermis cells in the described matrix usually afford the best results.

Application studies have shown that irritated skin is soothed on treatment with the matrix according to the present invention. In this regard, it is particularly advantageous if the collagen, chitosan and glycosylaminoglycan ingredients for use in the preparation of the present invention are employed in a balanced ratio to one another, especially as described in EP 296078. In other words, formation of a micro- or nanotubular aerogel and retention of this structure in a cosmetic preparation or skin covering will usually be possible only within certain ranges of ratios of the specific active ingredients.

In this case, the stationary biopolymer phase with the disperse phase(s) composed of physiological saline solution, minimal media or complete media is converted into a hydrogel phase. The matrix components of the present invention (i.e., collagen, chitosan, glycosylaminoglycan) result in an advantageous hydrogel phase. An individual active ingredient or only two of the active ingredients alone, or the combination of active ingredients in non-advantageous proportions do not result in desirable effects, for example, a favorable interaction with the cell culture media as aqueous phase, or with polyurethane or silicone matrices.

A preferred weight ratio of the collagen and chitosan components is from about 90 : 10 to about 60 : 40, in particular from about 85 : 15 to about 75 : 25.

Collagen is a designation for a family of long-fiber, linear-colloidal, high molecular weight scleroproteins of the extracellular matrix which occur in connective tissue (e.g. skin, cartilage, tendons, ligaments, blood vessels), in osseine (the protein-containing base substance of bone) and in dentin together with proteoglycans. They are regarded as the most common animal proteins in terms of quantity, with a proportion of 25-30 %. A mutual anchoring of the collagen fibers and of the cells is produced by fibronectin, which is able to bind collagen and other constituents of the extracellular matrix, but also becomes attached to receptors on cell surfaces. The composition of the collagens may vary depending on the origin. Collagens of types I to XIV are known, but only types I-III, V and XI have the described fiber structure.

When applied to the skin, advantages of the preparation of the present invention include: Supporting the regeneration process of the skin

Providing a optimum environment for the skin
Instrumental in improving the skin structure
Soothing of skin irritations
Improving the entire condition of the skin
Improving the appearance of the skin substantially
Helping the skin to regain its elasticity and healthy impression

The preparation (matrix) of the present invention may be a component of, by way of non-limiting example, aqueous gels, emulsions of the O/W, W/O/W or W/O type, microemulsions or cosmetic stick products and can thus be marketed in conventional cosmetic application forms.

In addition, the preparation of the present invention may be comprised in skin coverings, patches, pads, tissues or bandages.

An in-home-use application of the preparation of the present invention is possible, too. By way of non-limiting example, in the form of an aerogel, the matrix can be placed on the part of skin that is to be treated. The constituents of the matrix of the invention are biological polymers which can, through a specific mixing ratio, be converted into a stable aerogel and can even be reconstituted as stable hydrogel. Advantages were found and demonstrated for the preparation of the present invention for cell regeneration and proliferation of primary skin cells of the keratinocyte and fibroblast type. It is also possible through the glycosylaminoglycan, chitosan and collagen matrix interacting with the skin cells, in particular in the 3-D skin models, to induce the production of elastin, fibrillin and further biomarkers which are responsible for the quality of a healthy skin. It further is possible, through the interaction of matrix molecules, as described above, and the cell culture media, to markedly improve the reticular interlocking of the epidermis in the dermis. It is thus possible, through the preparation of the invention in interaction with the culture media, to achieve ideal regeneration of complete skin from only a few skin cells, and supply a pre-existing skin with the ideal healthy growth environment and nutrient factors. In interaction with polyurethane components, skin regeneration can be optimized under semi- or occlusive conditions, which helps to normalize in particular, keloids and other scars.

The process of producing the described matrix may advantageously comprise the addition of optionally acetylated chitosan to a collagen/water solution which also comprise cell culture media, and the subsequent addition of a glycosylaminoglycan, preferably at least chondroitin 4-sulfate and/or chondroitin 6-sulfate.

The preferred proportions of the active ingredients of the preparation according to the present invention allow sustained or controlled release of active agents such as, e.g., Q10, retinol, AHA (alpha-hydroxy acids), etc. and, in addition, may reduce or eliminate the side effects of these agents.

The most important alpha-hydroxy acids include glycolic acid, lactic acid, citric acid, tartaric acid, malic acid and salicylic acid. These acids contribute to an ablation of keratinized furfur. The skin immediately becomes smoother, fresher and softer. Pigmental moles become lighter.

Coenzyme Q10 or ubiquinone is present in almost all organisms and plays an important role in cell metabolism. Q10 also is an effective antioxidant, scavenges free radicals and stabilizes cell membranes. Thereby Q10 keeps the cells intact, functional and alive.

The term "skin cell culture medium" is intended to encompass all liquid, powdered or solid media in or on which individual cells can multiply or be cultivated. Those of skill in the art distinguish between purifying media such as, e.g., phosphate-buffered saline solution, minimal maintenance media and so-called complete media, in which cells are healthy and metabolically active. Complete media may be provided with growth factors from animal serum or so-called synthetic serum substitutes in order to improve the growth of specific cells or make same possible at all. For every cell type, but especially the culture of primary cells, there are media and media blends which support the growth, differentiation or metabolism of specific cells particularly well.

The combination according to the present invention of collagens, chitosans and glycosylaminoglycans (e.g., chondroitin sulfates) may be blended with all purifying, minimal or complete media, but particularly advantageously with complete media which have been composed for culturing skin cells and serve in particular as nutrient media for primary human fibroblasts and keratinocytes and which, as nutrient media, make it possible for the dermis to be regenerated from individual fibroblasts, or for the epidermis to be regenerated from individual keratinocytes. The skin cell culture media which are employed according to the present invention are thus particularly suitable for cultivating skin cells. Particular preference is given to skin cell culture media which are described as being suitable, in the composition of their individual ingredients, for the following purposes:
culture of fibroblast cells
culture of keratinocyte cells
co-cultures of keratinocytes and fibroblasts
co-cultures of fibroblasts/keratinocytes and further skin-relevant cells such as immune cells, melanocytes etc.
culture media for generating three-dimensional skin models.

The media which are preferably employed according to the present invention can act on keratinocyte/fibroblast mixed cultures and 3-D skin models.

It has surprisingly been found that cosmetic compositions which comprise the mixture according to the present invention of biomolecules and skin cell culture media are able in or on the human skin itself to activate or simulate the mechanisms which the skin uses for homeostasis and healthy autopoiesis. In this regard, the mixtures of fibroblast- or keratinocyte-relevant growth media may be employed directly or in suitable vesicle technologies, and may be used for medical/pharmaceutical purposes and cosmetic purposes.

In particular, so-called serum-free media have proved to be advantageous when the cell fraction of the primary keratinocytes and primary fibroblasts is to be positively influenced in the sense of optimized homeostasis.

The use of the skin-relevant culture media in interaction with the matrix biomolecules brings about autologous, healthy and individual regeneration of deficient skin functions *in vitro, ex vivo* and *in vivo.* It is thus possible for regeneration of the skin, skin tautness or else simply only the contribution to skin care to be significantly improved.

In principle, all skin cell culture media are suitable for use in cosmetic preparations. Particularly suitable skin cell culture media are those employed in the literature for cultivating skin cells or skin-relevant cells, for treating skin irritations and burns. In particular, media for cultivating remaining cells after extensive burns show an extremely advantageous effect after application of the topical preparations.

Skin cell culture media which are particularly advantageous according to the present invention include media which permit neogenesis of fibroblasts or keratinocytes alone or in mixed cultures and/or which reduce the formation and passaging of non-benign cells.

The skin cell culture media DMEM/HAM F12 (1:1) and MCDB 153 are particularly suitable for use in the present cosmetic preparations.

According to Barnes D. and Sato G., Anal. Biochem. 102, 255 [1980], DMEM/HAM F12 (1:1) is a 1:1 mixture where the nutrient content of HAM F12 medium is increased through addition of Dulbecco's MEM (DMEM = Dulbecco's Modified Eagles Medium). This medium is the basis for cultivating cell lines for human proteins such as, for example, erythropoetin.

DMEM/HAM F12 (1:1) medium has the following composition (in mg/L):

| | | | |
|---|---|---|---|
| NaCl | 6999.5 | L-Leucine | 59 |
| KCl | 311.8 | L-Lysine HCl | 91.25 |
| Na₂HPO₄ | 71 | L-Methionine | 17.24 |
| NaH₂PO₄-H₂O | 62.5 | L-Phenylalanine | 35.5 |
| MgSO₄-7H₂O | 100 | L-Proline | 17.25 |
| MgCl₂-6H₂O | 61 | L-Serine | 26.25 |
| CaCl₂ | 116.61 | L-Threonine | 53.5 |
| Fe(NO₃)₃-9H₂O | 0.05 | L-Tryptophan | 9 |
| FeSO₄-7H₂O | 0.417 | L-Tyrosine | 38.7 |
| CuSO₄-5H₂O | 0.00125 | L-Valine | 52.85 |
| ZnSO₄-7H₂O | 0.432 | | |
| D-Glucose | 3151 | Choline chloride | 9 |
| NaHCO₃ | 2438 | α-Biotin | 0.00365 |
| Na Pyruvate | 55 | Folic acid | 2.65 |
| Phenol red | 12.5 | D-Ca pantothenate | 2.24 |
| myo-Inositol | 12.6 | | |
| L-Alanine | 4.5Nicotinamide | 2.02 | |
| L-Arginine HCl | 147.5 | Pyridoxcal HCl | 2 |
| L-Asparagine-H₂O | 7.5Pyridoxine HCl | 0.031 | |
| L-Aspartic acid | 6.65 | Riboflavin | 0.22 |
| L-Cysteine HCl | 15.75 | Thiamine HCl | 2.17 |
| L-Cystine | 24 | Vitamin B₁₂ | 0.68 |
| L-Glutamine | 365.3 | Hypoxanthin | 2.05 |
| L-Glutamic acid | 7.35 | Thymidine | 0.37 |
| Glycine | 18.75 | Lipoic acid | 0.11 |
| L-Histidine HCl-H₂O | 31.5 | Linoleic acid | 0.042 |
| L-Isoleucine 54.5 | | Putrescine 2HCl | 0.081 |

According to Barnes D. and Sato G., Anal. Biochem. 102, 255 [1980], MCDB 153 medium is employed for cultivating human keratinocytes. Further, as minimal medium PBS, phosphate-buffered saline, with pH values of from 3.5 to 8.

MCDB 153 medium has the following composition (mg/L):

| | | | |
|---|---|---|---|
| NaCl | 7599 | Choline chloride | 13.96 |
| KCI | 111.83 | Putrescine | 0.1611 |
| Sodium acetate-3H₂O | 500 | Vitamin B₁₂ | 4.07 |
| Na₂HPO₄-7H₂O | 536.2 | Biotin | 0.0146 |
| MgCl₂-6H₂O | 122 | Calcium pantothenate | 0.258 |
| CaCl₂-2H₂O | 4.411 | Nicotinamide | 0.03663 |
| Glucose | 1081 | Pyridoxine HCl | 0.06171 |
| Sodium pyruvate | 55 | Thiamine HCl | 0.3373 |
| NaHCO₃ | 1176 | Adenine | 24.32 |
| Phenol red | 1.317 | myo-Inositol | 18.02 |
| HEPES | 6600 | Lipoic acid | 0.2063 |
| Thymidine | 0.7266 | | |
| L-Alanine | 8.91 | Folic acid | 0.79 |
| L-Arginine-HCl | 210.7 | Riboflavin | 0.03764 |
| L-Asparagine | 15.01 | | |
| L-Aspartic acid | 3.99 | CuSO₄-5H₂O | 0.0002496 |
| L-Cysteine HCl-H₂O | 42.04 | FeSO₄-7H₂O | 1.39 |
| L-Glutamine | 877.2 | MnSO₄-5H₂O | 0.000241 |
| L-Glutamic acid | 14.71 | (NH₄)₆Mo₇O₂₄ - 4H₂O | 0.001236 |
| Glycine | 7.51 | NiCl₂-6H₂O | 0.0001188 |
| L-Histidine HCl-H₂O | 16.77 | H₂SeO₃ | 0.003869 |
| L-Isoleucine | 1.968 | Na₂SiO₃-9H₂O | 0.1421 |
| L-Leucine | 65.6 | SnCl₂-2H₂O | 0.0001128 |
| L-Lysine-HCl | 18.27 | NH₄VO₃ | 0.000585 |
| L-Methionine | 4.476 | ZnSO₄-7H₂O | 0.144 |
| L-Phenylalanine | 4.956 | | |
| L-Proline | 34.53 | | |
| L-Serine | 63.06 | | |
| L-Threonine | 11.91 | | |
| L-Tryptophan | 3.06 | | |
| L-Tyrosine | 2.718 | | |
| L-Valine | 35.13 | | |

The advantage of the DMEM/HAM F12 (1:1) and MCDB 153 media is that they are particularly selected and suitable in cosmetic preparations for the cultivation of monolayer, two-dimensional and organotypical skin models, and permit the *in vitro* and *ex vivo* stimulation and/or retention of skin-specific biofunctions.

Additionally, it may be advantageous to add to the media solutions of the following compositions A and B as serum substitutes:

| Solution A | | Solution B | |
|---|---|---|---|
| Components (1000x) | µM | Components (1000x) | µM |
| FeSO₄-7H₂O | 3000 | Insulin human in 0.01 M HCl | 86 |
| ZnSO₄-7H₂O | 3000 | | |
| CoCl₂-6H₂O | 1000 | | |
| CuSO₄-5H₂O | 10 | | |
| Na₂SeO₃ | 10 | | |
| AlCl₃-6H₂O | 5 | | |
| CrK(SO₄)₂-12H₂O | 1.4 | | |
| NiCl₃-6H₂O | 1 | | |
| MnCl₂-4H₂O | 1 | | |
| EDTA.Nₐ2-2H₂O | 30000 | | |
| Polysorbate 80 VG | 3820 | | |

According to the literature, the liquid media are usually prepared by using high-purity, pyrogen-free water. This water complies with the WFI quality (water for injection) of Pharmacopeia Europa. The liquid media are sterilized by filtration and bottled, the systems and methods of manufacture being such that entry of endotoxins and microbes is largely precluded.

The media that are preferred for use in the present invention show advantageous properties in relation to skin regeneration even if the media compositions are altered, such as, for example, with or without choline chloride, with or without H₂SeO₃.

The skin cell culture media and the mixture of biomolecules (collagen/chitosan/- glycosylaminoglycan) and additives may advantageously be mixed into a cosmetic preparation in a proportion of up to 99.9 % by weight, based on the total weight of the preparation.

Some of the advantages associated with the preparation of the present invention which comprises a combination of certain components of cell culture media with the active ingredients collagen, chitosan and glycosylaminoglycan are illustrated in the Examples below.

In the present specification and the appended claims, cosmetic preparations or matrices are intended to include topical preparations which are suitable for applying said media to the skin in fine distribution and preferably in a form which can be absorbed through the skin. Examples of application forms which are suitable for this purpose include aqueous and hydroalcoholic solutions, sprays, foams, foam aerosols, ointments, aqueous gels, emulsions of the O/W or W/O type, microemulsions, hydrophilic or lipophilic patches and cosmetic stick products. Particularly suitable carriers include aqueous gels, O/W emulsions, W/O/W emulsions and microemulsions. The preparation can also be used, for example, in body-cleansing compositions such as, e.g., soaps, shower baths, shampoos and the like.

Preferred cosmetic formulations include hydrogels and emulsions of any type, in particular O/W emulsions.

All lipids known for use in cosmetics can, for example, be employed as oily or lipid phase.

Preparations of the present invention in the form of an emulsion will usually comprise one or more emulsifiers. These emulsifiers may advantageously be chosen from nonionic, anionic, cationic and amphoteric emulsifiers.

Besides water and physiologically suitable solvents, it is possible to use, *inter alia,* care constituents, oils, waxes, fats, refatting substances, thickeners, antioxidants, emulsifiers, substances suitable as sunscreen filters, enzymes, amino acids, proteins, polysaccharides and/or fragrances. According to the invention, apart from the aforementioned substances the preparations may optionally also comprise the additives that are customary in cosmetics, for example perfume, dyes, antimicrobial substances, refatting agents, complexing and sequestering agents, pearlescent agents, plant extracts, vitamins, active ingredients, preservatives, bactericides, coloring pigments, thickeners, emollients, moisturizers and/or humectants, and other usual ingredients of a cosmetic or dermatological formulation such as, e.g., alcohols, polyols, polymers, foam stabilizers, electrolytes, organic solvents and silicone derivatives.

Non-limiting examples of advantageous additives include specific active ingredients such as, for example, antioxidants. These antioxidants may advantageously be selected from amino acids (e.g. glycine, lysine, arginine, cysteine, cystine, histidine, tyrosine, tryptophan) and derivatives thereof (as salt, ester, ether, sugar, nucleotide, nucleoside, peptide and lipid compound), imidazoles (e.g., urocanic acid) and derivatives thereof (e.g., as salt, ester, ether, sugar, nucleotide, nucleoside, peptide and/or lipid compound), peptides such as D,L-carnosine, D-carnosine, L-carnosine, anserine and derivatives thereof (e.g., as salt, ester, ether, sugar, thiol, nucleotide, nucleoside, peptide and lipid compound), carotenoids, carotenes (e.g. α-carotene, p-carotene, ψ-lycopene, phytoene,) and derivatives thereof (e.g., as salt, ester, ether, sugar, nucleotide, nucleoside, peptide and/or lipid compound), chlorogenic acid and derivatives thereof (e.g., as salt, ester, ether, sugar, thiol, nucleotide, nucleoside, peptide and/or lipid compound), aurothioglucose, propylthiouracil and other thiols (e.g., thioredoxin, lipoic acid, glutathione, cysteine, cystine, cystamine and their glycosyl, N-acetyl, methyl, ethyl, propyl, amyl, butyl and lauryl, palmitoyl, oleyl, γ-linoleyl, cholesteryl and glyceryl esters) and the salts thereof, dilauryl thiodipropionate, distearyl thiodipropionate, thiodipropionic acid and derivatives thereof (e.g., as salt, ester, ether, sugar, thiol, nucleotide, nucleoside, peptide and/or lipid compound) and sulfoximine compounds (e.g., homocysteine sulfoximine, buthionine sulfones, penta-, hexa-, heptathio-nine sulfoximine) in very low tolerated dosages (e.g. pmol to µmol/kg). Also included are (metal) chelators (e.g., apoferritin, desferral, lactoferrin, α-hydroxy fatty acids, palmitic acid, phytic acid) and derivatives thereof (e.g., as salt, ester, ether, sugar, thiol, nucleotide, nucleoside, peptide and/or lipid compound), α-hydroxy acids (e.g. citric acid, lactic acid, malic acid), humic acid, bile acid, bile extracts, bilirubin, biliverdin, EDTA, EGTA and derivatives thereof, unsaturated fatty acids and derivatives thereof (e.g., γ-linolenic acid, linoleic acid, oleic acid), folic acid and derivatives thereof, furfurylidenesorbitol and derivatives thereof, ubiquinone, ubiquinol, plastoquinone and derivatives thereof (e.g., as salt, ester, ether, sugar, thiol, nucleotide, nucleoside, peptide and lipid compound), vitamin C and derivatives (e.g., ascorbyl palmitate, Mg ascorbyl phosphate, ascorbyl acetate), tocopherols and derivatives (e.g., vitamin E acetate), and phenolic compounds and plant extracts containing same such as, for example, flavonoids (e.g., glycosyl rutin, ferulic acid, caffeic acid), furfurylidene glucitol, butylated hydroxytoluene, butylated hydroxyanisole, nordihydroguaiaretic resin acid, nordihydroguaiaretic acid, trihydroxybutyrophe-none and derivatives thereof (as salt, ester, ether, sugar, nucleotide, nucleoside, peptide and lipid compound), uric acid and derivatives thereof, mannose and derivatives thereof (e.g., as salt, ester, ether, sugar, thiol, nucleotide, nucleoside, peptide and lipid compound), zinc and its derivatives (e.g., ZnO, ZnSO₄) selenium and its derivatives (e.g., selenomethionine, ebselen), stilbenes and derivatives thereof (e.g., stilbene oxide, trans-stilbene oxide) and the derivatives (e.g., the salt, ester, ether, sugar, thiol, nucleotide, nucleoside, peptide and/or lipid compounds) of these active ingredients which are suitable according to the invention.

The additional use of a buffer may sometimes become necessary for stabilizing the ingredients of the disperse phase. In this regard, phosphate-buffered saline solutions and citrate buffers are examples of preferably employed buffers.

Besides antioxidants, combinations of the preparations of the present invention with specific ingredients which are preferably chosen from Q10, AGR, Zn orotate, carnitine, creatine and/or taurine are particularly preferred.

AGR (alpha-glucosyl rutin) belongs to the flavonoids which are found in most plants. AGR is capable of protecting the cells of the intrinsic immune system of the skin from environmental damage such as, e.g., from UV radiation.

Areas of application of the preparation of the present invention which have proved to be particularly advantageous include the care of all skin types with the exception of all septic inflammations, and also special applications such as microdermal abrasion, acid peeling and retinol treatments. The skin regeneration and soothing of the skin by the preparations of the present invention is evident in these cases.

An additional preferred field of application of the preparation of the present invention is cosmetic care of the skin, in particular for beautification.

The packaging for the preparation of the present invention can include all cosmetically customary dosage systems such as, e.g., jars, pump bottles, pipette bottles, cartridges or capsules.

For problematic formulations into which the cell medium cannot be incorporated, there is the possibility to mix the cell culture media and cosmetic product only shortly before use, through special packaging elements such as, for example, double cartridges with a mixing head as known, for example, from 2-component adhesives. The packaging of the cell culture medium may also be designed for refilling, so that only fresh product is used.

It also is advantageous to incorporate the matrix of the invention in a polyurethane matrix and configure the resultant product as cosmetic skin covering, or as pad. Non-limiting examples of polyurethane matrices which are suitable for these purposes include those which are described in DE 42 33 289, DE 43 08 347, DE 43 08 445, DE 43 28 190 and DE 101 28 685.

Advantageous exemplary embodiments of the present invention follow. Unless indicated otherwise, the quantitative data are based on weight %. It is possible in all the preparations for the ratio of the matrix molecules collagen, chitosan and glycosylaminoglycan to be from about 0.00001 % by weight to about 99 % by weight of the final formulation, preferably from anbout 0.0005 % by weight to about 50% by weight and ideally from about 0.0015 % to about 30 % by weight, based on the total weight of the preparation. The dispersant "culture medium" preferably corresponds to an osmotic pressure of an about 0.5 % to about 2% sodium chloride solution, but ideally corresponds to the physiological osmotic pressure of human tissue, especially of the skin.

### Examples

### Example 5 O/W Emulsion

| | |
|---|---|
| KERATINOCYTE MEDIUM MCDB153 | 40% by weight |
| COLLAGEN/CHITOSAN/CHONDROITIN SULFATE matrix | 6% by weight |
| plus, blended in any proportion: | |
| WATER (AQUA) | |
| GLYCERIN | |
| HYDROGENATED COCO-GLYCERIDES | |
| SQUALANE | |
| GLYCERYL STEARATE CITRATE | |
| CAPRYLIC/CAPRIC TRIGLYCERIDE | |
| ETHYLHEXYL COCOATE | |
| MYRISTYL ALCOHOL | |
| BUTYROSPERMUM PARKII (SHEA BUTTER) | |
| BUTYLENE GLYCOL | |
| CETYL ALCOHOL | |
| TOCOPHERYL ACETATE | |
| PHENOXYETHANOL | |
| SODIUM CHLORIDE | |
| IMIDAZOLIDINYL UREA | |
| CARBOMER | |
| XANTHAN GUM | |
| METHYLPARABEN | |
| EDTA | |
| SODIUM HYDROXIDE | |
| BHT | |
| ETHYLPARABEN | |
| BUTYLPARABEN | |
| ISOBUTYLPARABEN | |
| PROPYLPARABEN | |

### Example 6 W/O/W Emulsion

| | % by weight |
|---|---|
| PEG-100 stearate | 2.00% |
| Glyceryl stearate | 4.00% |
| Squalane | 1.50% |
| Squalene | 1.50 % |
| Isopropyl palmitate | 5.40 % |
| MCDB 153/DME 1:1 | 0.360 % |
| Magnesium sulfate | 0.240% |
| Preservative | 0.50 % |
| COLLAGEN/CHONDROITIN SULFATE/CHITOSAN | 5% |
| Water VES ad | 100.00 |

The fatty phase containing the emulsifier is heated to 80°C. The aqueous phase without the part that contains the medium is heated to 80°C as well. The two phases are combined at 80°C, homogenized for about 3-10 minutes and then cooled to 48°C or room temperature. Then, keeping the temperature constant to ± 1 °C, the part of the aqueous phase which contains the medium is added and mixed.

### Example 7

| | |
|---|---|
| PEG-40 stearate | 1.00 % |
| Glyceryl stearate | 2.00 % |
| Cetyl alcohol | 3.00 % |
| Mineral oil DAB 9 | 2.00 % |
| Safflower oil | 2.00 % |
| Isopropyl palmitate | 4.50 % |
| Glycerin | 3.00 % |
| Magnesium sulfate | 1.20 % |
| Preservative | 0.50 % |
| Collagen/chitosan/glycosylaminoglycan matrix | 2.00 % |
| Deionized water | ad 100.00 % |
| of which DMEM/HAM F12 (1:1) | 2.5 % |

### Example 8

| | |
|---|---|
| PEG-80 stearate | 2.00 % |
| Cetyl alcohol | 3.00 % |
| Mineral oil DAB 9 | 1.50 % |
| Evening primrose oil | 2.50 % |
| Isopropyl palmitate | 5.40 % |
| Propylene glycol | 3.00 % |
| Potassium chloride | 0.60 % |
| Preservative | 0.50 % |
| Collagen/chitosan/gycosylaminoglycan matrix | 1.50 % |
| Water VES | ad 100.00 % |
| of which DMEM/HAM F12 (1:1) | 5 % |

### Example 9

| | |
|---|---|
| Steareth-100 | 2.00 % |
| Myristyl alcohol | 1.00 % |
| Mineral oil DAB 9 | 3.00 % |
| Castor oil | 3.00 % |
| Cyclomethicone | 2.00 % |
| Propylene glycol | 3.00 % |
| Glycerin | 5.00 % |
| Potassium chloride | 3.00 % |
| Collagen/chitosan/glycosylaminoglycan matrix | 4.50% |
| Preservative | 0.50 % |
| Water VES | ad 100.00 % |
| of which MCDB 153 | 0.5 % |

### Example 10

| | |
|---|---|
| Steareth-20 | 2.00 % |
| Cetearyl | 3.00 % |
| Petrolatum | 0.50 % |
| Wheat germ oil | 1.50 % |
| Dimethicone | 5.00 % |
| Glycerin | 5.00 % |
| Sodium chloride | 3.00 % |
| Preservative | 0.50% |
| PUR | 1.50 % |
| Collagen/chitosan/glycosylaminoglycan matrix | 5.65 % |
| Water VES | ad 100.00 % |
| of which DMEM/HAM F12 (1:1) | 15% |

### Example 11

| | |
|---|---|
| Dimethicone copolyol | 2.00% |
| Cetearyl alcohol | 3.00 % |
| Petrolatum | 0.50 % |
| Wheat germ oil | 1.50 % |
| Dimethicone | 5.00 % |
| Glycerin | 5.00 % |
| Sodium chloride | 3.00 % |
| Preservative | 0.50 % |
| Collagen/chitosan/glycosylaminoglycan matrix | 6.05 % |
| Water VES | ad 100.00 % |
| of which MCDB 153 | 1.5 % |

### Example 12

| | |
|---|---|
| PEG-20 behenate | 2.00 % |
| Stearyl alcohol | 3.00 % |
| Petrolatum | 1.00 % |
| Grape seed oil | 3.00 % |
| Dimethicone | 3.00 % |
| Sorbitol | 5.00 % |
| Zinc sulfate | 3.00 % |
| Preservative | 0.50 % |
| Collagen/chitosan/glycosylaminoglycan matrix | 10.5 % |
| Water VES | ad 100.00 % |
| of which MCDB 153 | 5% |

### Example 13

| | |
|---|---|
| Decaglyn 1-IS | 2.00 % |
| Stearyl alcohol | 3.00 % |
| Petrolatum | 1.00 % |
| Grape seed oil | 3.00 % |
| Dimethicone | 3.00 % |
| Sorbitol | 5.00 % |
| Zinc sulfate | 3.00 % |
| Preservative | 0.50 % |
| Collagen/chitosan/glycosylaminoglycan matrix | 25.00 % |
| Water VES | ad 100.00 % |
| of which MCDB 153 | 40% |

### Example 14

| | |
|---|---|
| PEG-20 myristate | 2.00 % |
| Stearyl alcohol | 3.00 % |
| Petrolatum | 2.00 % |
| Castor oil | 5.00 % |
| Dimethicone | 5.00 % |
| Sorbitol | 5.00 % |
| Zinc sulfate | 3.00 % |
| Preservative | 0.50 % |
| Collagen/chitosan/glycosylaminoglycan matrix | 3.5 % |
| Water VES | ad 100.00 % |
| of which MCDB 153/RPMI 1640/serum substitutes | 0.1 % |

### Example 15

| | |
|---|---|
| Sucrose laurate | 2.00% |
| Stearyl alcohol | 3.00% |
| Petrolatum | 2.00 % |
| Castor oil | 5.00 % |
| Dimethicone | 5.00 % |
| Sorbitol | 5.00 % |
| Zinc sulfate | 3.00 % |
| Preservative | 0.50 % |
| Collagen/chitosan/glycosylaminoglycan matrix | 8.50 % |
| Water VES | ad 100.00 % |
| of which MCDB 153 | 12% |

### Example 16

| | |
|---|---|
| PEG-80 behenate | 2.00 % |
| Glyceryl behenate | 4.00 % |
| Squalane | 3.00 % |
| Castor oil | 5.40 % |
| Glycerin | 6.00 % |
| Magnesium sulfate | 2.60 % |
| Preservative | 0.50 % |
| Collagen/chitosan/glycosylaminoglycan matrix | 0.0655 |
| Water VES | ad 100.00 % |
| of which MCDB 153/DME | 8.5 % |

### Example 17 (O/W Emulsion):

| | |
|---|---|
| Glyceryl stearate citrate | 3.00 % |
| Stearyl alcohol | 1.00 % |
| Octyldodecanol | 1.00 % |
| Caprylic/capric triglyceride | 1.00 % |
| Dicaprylyl ether | 1.00 % |
| Carbomer | 0.15 % |
| Glycerin | 3.00 % |
| Perfume, preservative, NaOH | |
| dyes, antioxidants, etc. | q.s. |
| Collagen/chitosan/glycosylaminoglycan matrix | 4.0 % |
| Water | ad 100.00 % |
| of which DMEM/HAM's F-12 (1:1) | 2.5 % |
| pH adjusted to | 5.5 |

### Example 18 (O/W Emulsion):

| | |
|---|---|
| Glyceryl stearate citrate | 3.00 % |
| Stearyl alcohol | 1.00 % |
| Octyldodecanol | 0.25 % |
| Caprylic/capric triglyceride | 0.25 % |
| Dicaprylyl ether | 0.25 % |
| Carbomer | 0.15 % |
| Glycerin | 3.00 % |
| Perfume, preservative, NaOH dyes, antioxidants, etc. | q.s. |
| Collagen/chitosan/glycosylaminoglycan matrix | 5.0 % |
| Water | ad 100.00 % |
| of which MCDB 153 | 0.5 % |
| pH adjusted to | 5.5 |

### Example 19 (O/W Emulsion):

| | |
|---|---|
| Glyceryl stearate citrate | 3.00 % |
| Behenyl alcohol | 1.00 % |
| Dimethicone | 1.50 % |
| Cycolmethicone | 1.50 % |
| Carbomer | 0.15 % |
| Glycerin | 6.00 % |
| Perfume, preservative, NaOH dyes, antioxidants, etc. | q.s. |
| Collagen/chitosan/glycosylaminoglycan matrix | 7.5 % |
| Water | ad 100.00 |
| of which DMEM/HAM's F-12 (1:1) | 5% |
| pH adjusted to | 5.5 |

### Example 20 (O/W Emulsion):

| | |
|---|---|
| Glyceryl stearate citrate | 3.00 % |
| Stearyl alcohol | 1.00 % |
| Octyldodecanol | 0.25 % |
| Caprylic/capric triglyceride | 0.25 % |
| Dicaprylyl ether | 0.25 % |
| Dimethicone | 0.50 % |
| Carbomer | 0.15 % |
| Glycerin | 3.00 % |
| Aluminum starch octenyl succinate | 0.50 % |
| Talc | 0.50 % |
| Bentonite | 0.10 % |
| Collagen/chitosan/glycosylaminoglycan matrix | 0.40 % |
| Perfume, preservative, NaOH dyes, antioxidants etc. | q.s. |
| Water | ad 100.00 |
| of which MCDB 153 | 80 % |
| pH adjusted to | 5.5 |

### Example 21 (O/W Emulsion):

| | |
|---|---|
| Glyceryl stearate citrate | 3.00 % |
| Cetyl alcohol | 1.00 % |
| Squalane | 1.00 % |
| Jojoba oil | 1.00 % |
| Liquid paraffin | 1.00 % |
| Carbomer | 0.10 % |
| Glycerin | 3.00% |
| Serine | 0.50% |
| Tocopherol acetate | 1.00 % |
| Carbomer | 0.10 % |
| Xanthan gum | 0.10% |
| Collagen/chitosan/glycosylaminoglycan matrix | 1.0 % |
| Perfume, preservative, NaOH dyes, antioxidants etc. | q.s. |
| Water | ad 100.00 |
| of which MCDB 153 | 75.5 % |
| pH adjusted to | 6.0 |

### Example 22 (O/W Emulsion):

| | |
|---|---|
| Glyceryl stearate citrate | 3.00 % |
| Cetyl alcohol | 0.50 % |
| Octyldodecanol | 0.40 % |
| Caprylic/capric triglyceride | 0.40 % |
| Dicaprylyl ether | 0.40 % |
| Carbomer | 0.10 % |
| Glycerin | 3.00 % |
| Serine | 0.50 % |
| Collagen/chitosan/glycosylaminoglycan matrix | 1.2 % |
| Perfume, preservative, NaOH dyes, antioxidants etc. | q.s. |
| Water | ad 100.00 |
| of which MCDB 153 | 45.5 % |
| pH adjusted to | 5.5 |

### Example 23 (Emulsion Make Up):

| | |
|---|---|
| Glyceryl stearate citrate | 3.00 % |
| Stearyl alcohol | 1.00 % |
| Dimethicone | 0.50 % |
| Glycerin | 1.50 % |
| 1,3 Butylene glycol | 1.50 % |
| Magnesium silicate | 1.00 % |
| Mica | 1.00 % |
| Iron oxides | 1.00 % |
| Titanium dioxide | 2.50 % |
| Talc | 5.00 % |
| Carbomer | 0.15 % |
| Collagen/chitosan/glycosylaminoglycan matrix | 0.50 % |
| Perfume, preservative, NaOH, dyes, antioxidants etc. | q.s. |
| Water | ad 100.00 |
| of which MCDB 153/Ham's F12/RPMI 1640 | 0.5 % |
| pH adjusted to | 5.5 |

### Example 24 (O/W Emulsion):

| | |
|---|---|
| Glyceryl stearate citrate | 3.00 % |
| Stearyl alcohol | 1.00 % |
| Octyldodecanol | 0.25 % |
| Caprylic/capric triglyceride | 0.25 % |
| Dicaprylyl ether | 0.25 % |
| Octyl methoxycinnamate | 4.00 % |
| Benzophenone-3 | 3.00 % |
| Octyl salicylate | 3.00 % |
| Carbomer | 0.15 % |
| Glycerin | 3.00 % |
| Collagen/chitosan/glycosylaminoglycan matrix | 1.75 % |
| Perfume, preservative, NaOH dyes, antioxidants etc. | q.s. |
| Water | ad 100.00 |
| of which MCDB 153 | 40 % |
| pH adjusted to | 5.5 |

### Example 25 (O/W Emulsion):

| | |
|---|---|
| Glyceryl stearate citrate | 3.00 % |
| Stearyl alcohol | 1.00 % |
| Octyldodecanol | 0.50 % |
| Caprylic/capric triglyceride | 0.50 % |
| Dicaprylyl ether | 0.50 % |
| Distarch phosphate | 1.00 % |
| Ethanol | 10.00 % |
| Carbomer | 0.15 % |
| Glycerin | 3.00 % |
| Collagen/chitosan/glycosylaminoglycan matrix | 0.0625 % |
| Perfume, preservative, NaOH dyes, antioxidants etc. | q.s. |
| Water | ad 100.00 |
| of which MCDB 153 | 35 % |
| pH adjusted to | 5.5 |

### Example 26 (Emulsifier Gel):

| | |
|---|---|
| Glyceryl stearate citrate | 3.00 % |
| Stearyl alcohol | 1.00 % |
| Ethanol | 2.00 % |
| Aluminum starch octenyl succinate | 0.25 % |
| Talc | 0.25 % |
| Tapioca starch | 0.25 % |
| Carbomer | 0.15 % |
| Glycerin | 3.00 % |
| Collagen/chitosan/glycosylaminoglycan matrix | 0.10 % |
| Perfume, preservative, NaOH dyes, antioxidants etc. | q.s. |
| Water | ad 100.00 |
| of which DMEM/HAM's F-12 (1:1) | 3.5 % |
| pH adjusted to | 5.5 |

### Example 27 Testing

To investigate the effect of the substances (d) used in the preparation of the present invention on the viability of human normal fibroblasts the following testing was carried out.

A test sample according to the present invention of the following composition was prepared, filtered through a 0.22 µm filter and diluted in a Fetal Calf Serum (FCS) depleted cell culture media F2 (DMEM/HAM F12 (1:1) containing 2% of FCS and antibiotics) at concentrations of 1 %, 2 %, 3 % and 5 % (w/w).

| Components (INCI/CTFA adopted names) | % w/w |
|---|---|
| Water (Aqua) | 98.2691 |
| Butylene Glycol | 0.5 |
| Soluble Collagen | 0.41 |
| Phenoxyethanol | 0.375 |
| Chitosan | 0.11 |
| Sodium Chloride | 0.064 |
| Methylparaben | 0.05 |
| Sodium Chondroitin Sulfate | 0.046 |
| Glucose | 0.045 |
| Butylparaben | 0.04 |
| Propylparaben | 0.02 |
| Lysine Hydrochloride | 0.0146 |
| Isobutylparaben | 0.01 |
| Threonine | 0.0095 |
| Arginine | 0.0084 |
| Ethylparaben | 0.005 |
| Histidine | 0.0042 |
| Serine | 0.0042 |
| Potassium Chloride | 0.004 |
| Glycine | 0.003 |
| Calcium Chloride | 0.0026 |
| Magensium Sulfate | 0.002 |
| Tryptophan | 0.0016 |
| Sodium Phosphate | 0.0014 |
| Folic Acid | 0.0004 |
| Calcium Panthothenate | 0.00004 |

In parallel a comparative test sample which consisted only of water, collagen, chitosan and sodium chondroitin sulfate in the same relative weight ratios as in the test sample according to the invention was prepared and diluted with F12 media to concentrations of 1 %, 2 %, 3 % and 5 % (w/w).

### Incubation Protocol

Human Normal Fibroplasts (P8) were cultured in F12 media and placed in a 24 well culture plate at a cell density of 20,000 cells/well in 100 µL of F2 media per well. In a controlled atmosphere the cells were incubated with 900 µL of test composition per well at 37°C for 24 hours and 48 hours. Pure F2 media was used as negative control (NC) and media which differs from F2 media by containing 10 % instead of 2 % of FCS was used as positive control (PC). All experiments wered conducted in tetraplicate (n = 4).

### Evaluation Protocol

The viability of the cells after the 24 and 48 hour incubation periods was evaluated by measuring their alkaline phosphatase activity. Specifically, the p-nitrophenyl phosphate (PNPP) reduction capacity of the incubated cells at pH 5 during 45 minutes was followed spectroscopically at a wavelength of 405 nm.

### Statistics

Data from the assays were expressed as mean +/- Standard Deviation (S.D.). The statistical significance between the groups was assessed by the student t test.

### Results

The following table lists the obtained results in terms % activation of the cells incubated with the various tested samples compared to cells that were incubated with the negative control (NC).

**TABLE: Cell Proliferation Results**

| **Tested Sample** | **Raw Data (mean)** | **Raw Data (S.D.)** | **% Activation vs. NC** |
|---|---|---|---|
| NC | 0.021 | 0.022 | 100 |
| PC | 0.023 | 0.020 | 110 |
| Invention 1% | 0.034 | 0.009 | 162 |
| Invention 2% | 0.048 | 0.003 | 229 |
| Invention 3% | 0.052 | 0.005 | 248* |
| Invention 5% | 0.062 | 0.005 | 295* |
| Comparative 1% | 0.026 | 0.015 | 124 |
| Comparative 2% | 0.030 | 0.005 | 143 |
| Comparative 3% | 0.009 | 0.006 | 43 |
| Comparative 5% | 0.027 | 0.022 | 129 |

| | | | |
|---|---|---|---|
| * significantly different from Negative Control (NC) group (p<0.01) | | | |

The data summarized in the above table shows that the substances of group (d) in combination with a collagen/chitosan/glycosylaminoglycan combination induce cell proliferation in a dose-related way. Cell proliferation was strongly and significantly improved when the test sample according to the invention was used at concentrations of 3 % and 5 % (w/w), with values of +248 % and + 295 % versus the negative control, respectively.

In comparison, the collagen/chitosan/glycosylaminoglycan combination alone (i.e., without the substances of group (d)) did not appear to be capable of significantly inducing cell proliferation, and even seemed to exhibit cytotoxicity when used at high concentrations.

## Claims

1. A cosmetic skin care method, wherein the method comprises applying to a healthy skin a cosmetic preparation which is obtainable by combining substances comprising
(a) at least one of collagen,
(b) at least one of chitosan and an acetyl derivative thereof with a degree of acetylation of up to 50 %.
(c) at least one of a glycosylaminoglycan and
(e) one or more skin cell culture media,
wherein the preparation comprises
(d) at last one substance selected from amino acids, α-biotin, (NH₄)₆Mo₇O₂₄, adenine, AlCl₃, biotin, CaCl₂, calcium pantothenate, choline chloride, CoCl₂, CrK(SO₄)₂. CuSO₄, D-Ca pantothenate, EDTA.Na₂, EDTA.Na₃, Fe(NO₃)₃, FeSO₄, folic acid, glucose, H₂SeO₃, HEPES, hypoxanthine, insulin human, KCI, linoleic acid, lipoic acid, MgCl₂, MnCl₂, MnSO₄, myo-inositol, Na₂HPO₄, Na₂SeO₃, Na₂SiO₃, NaCl, NaH₂PO₄, NaHCO₃, sodium pyruvate, sodium acetate, NH₄VO₃, NiCl₂, nicotinamide, phenol red, polysorbate 80, putrescine, putrescine 2HCl, pyridoxine HCl, pyridoxal HCl, riboflavin, SnCl₂, thiamine HCl, thymidine, vitamin B₁₂ and ZnSO₄,
to support the regeneration process of the skin, provide a optimum environment for the skin, improve the skin structure, improve the entire condition of the skin, improve the appearance of the skin and/or help the skin to regain its elasticity and healthy impression.

2. The method of claim 1, wherein the amino acids comprise one or more of L-alanine, L-arginine, L-asparagine. L-aspartic acid, L-cysteine, L-cystine, glycine, L-glutamine, L-glutamic acid, L-histidine, L-isoleucine, L-leucine, L-lysine, L-methionine, L-phenylalanine, L-proline, L-serine, L-threonine, L-tryptophan, L-tyrosine, L-valine and the hydrochloride salts thereof.

3. The method of claim 1, wherein at least one of substances (a) and (b) is of marine origin or of synthetic origin.

4. The method of claim 1, wherein the glycosylaminoglycan comprises at least one of chondroitin 4-sulfate and chondroitin 6-sulfate.

5. The method of claim 1, wherein a weight ratio of substances (a) and (c) is from 35 : 1 to 3 : 1.

6. The method of claim 1, wherein a weight ratio of substances (a) and (b) is from 10 : 1 to 1.5 1.

7. The method of claim 1, wherein a weight ratio of substances (b) and (c) is from 10 : 1 to 1 : 1.

8. The method of claim 1, wherein the skin cell culture media comprise at least one of DMEM/HAM F12 (1:1) and MCDB 153.

9. The method of claim 1, wherein the preparation further comprises a citrate buffer.

10. The method of claim 1, wherein the preparation further comprises at least one of Q10, alpha-glucosyl rutin, an alpha-hydroxy acid, Zn orotate, carnitine, creatine and taurine.

## Patentansprüche

1. Kosmetisches Hautpflegeverfahren, wobei das Verfahren umfasst, dass man ein Kosmetikpräparat, das dadurch erhältlich ist, dass man Substanzen umfassend
(a) mindestens eine aus Collagen,
(b) mindestens eine aus Chitosan und einem Acetylderivat mit einem Acetylierungsgrad von bis zu 50%,
(c) mindestens eine von einem Glycosylaminoglycan
und
(e) ein oder mehrere Hautzellkulturmedien,
wobei das Präparat Folgendes umfasst:
(d) mindestens eine Substanz, ausgewählt aus Aminosäuren, α-Biotin, (NH₄)₆Mo₇O₂₄, Adenin AlCl₃, Biotin, CaCl₂, Calciumpantothenat, Cholinchlorid, CoCl₂, CrK(SO₄)₂, CuSO₄, D-Ca-Pantothenat, EDTA.Na₂, EDTA.Na₃, Fe(NO₃)₃, FeSO₄, Folsäure, Glukose, H₂SeO₃, HEPES, Hypoxanthin, menschliches Insulin, KCl, Linolensäure, Liponsäure, MgCl₂, MnCl₂, MnSO₄, myo-Inosit, Na₂HPO₄, Na₂SeO₃, Na₂SiO₃, NaCl, NaH₂PO₄, NaHCO₃, Natriumpyruvat, Natriumacetat, NH₄VO₃, NiCl₂, Nicotinamid, Phenolrot, Polysorbat 80, Putrescin, Putrescin-2HCl, Pyridoxin-HCl, Pyridoxal-HCl, Riboflavin, SnCl₂, Thiamin-HCl, Thymidin, Vitamin B₁₂ und ZnSO₄,
kombiniert, auf gesunde Haut aufträgt, um den Regenerationsvorgang der Haut zu unterstützen, eine optimale Umwelt für die Haut bereitzustellen, die Hautstruktur zu verbessern, den Gesamtzustand der Haut zu verbessern, das Erscheinungsbild der Haut zu verbessern und/oder der Haut zu helfen, ihre Elastizität und ihren gesunden Eindruck wiederzuerlangen.

2. Verfahren nach Anspruch 1, wobei die Aminosäuren eine oder mehrere aus L-Alanin, L-Arginin, L-Asparagin, L-Asparaginsäure, L-Cystein, L-Cystin, Glycin, L-Glutamin, L-Glutaminsäure, L-Histidin, L-Isoleucin, L-Leucin, L-Lysin, L-Methionin, L-Phenylalanin, L-Prolin, L-Serin, L-Threonin, L-Tryptophan, L-Tyrosin, L-Valin und den Hydrochloridsalzen davon umfasst.

3. Verfahren nach Anspruch 1, wobei mindestens eine der Substanzen (a) und (b) marinen Ursprungs oder synthetischen Ursprungs ist.

4. Verfahren nach Anspruch 1, wobei das Glycosylaminoglycan mindestens eines von Chondroitin-4-sulfat und Chondroitin-6-sulfat umfasst.

5. Verfahren nach Anspruch 1, wobei ein Gewichtsverhältnis der Substanzen (a) und (c) von 35:1 bis 3:1 beträgt.

6. Verfahren nach Anspruch 1, wobei ein Gewichtsverhältnis der Substanzen (a) und (b) von 10:1 bis 1,5:1 beträgt.

7. Verfahren nach Anspruch 1, wobei ein Gewichtsverhältnis der Substanzen (b) und (c) von 10:1 bis 1:1 beträgt.

8. Verfahren nach Anspruch 1, wobei die Hautzellkulturmedien mindestens eines aus DMEM/HAM F12 (1:1) und MCDB 153 umfassen.

9. Verfahren nach Anspruch 1, wobei das Präparat weiterhin einen Citratpuffer umfasst.

10. Verfahren nach Anspruch 1, wobei das Präparat weiterhin mindestens eines aus Q10, alpha-Glucosylrutin, einer alpha-Hydroxysäure, Zn-Orotat, Carnitin, Kreatin und Taurin umfasst.

## Revendications

1. Méthode de soins cutanés cosmétiques, où la méthode comprend l'application à une peau saline d'une préparation cosmétique pouvant être obtenue par la combinaison de substances comprenant
(a) au moins l'un des membres du groupe constitué par le collagène,
(b) au moins l'un des membres du groupe constitué par le chitosane et un dérivé acétylé de celui-ci avec un degré d'acétylation pouvant atteindre 50 %,
(c) au moins l'un des membres du groupe constitué par un glycosylaminoglycane et
(e) un ou plusieurs milieux de culture de cellules cutanées,
où la préparation comprend
(d) au moins une substance choisie parmi les suivantes : acides aminés, α-biotine, (NH₄)₆Mo₇O₂₄, adénine, AlCl₃, biotine, CaCl₂, pantothénate de calcium, chlorure de choline, CoCl₂, CrK(SO₄)₂, CuSO₄, D-pantothénate de Ca, EDTA Na₂, EDTA.Na₃, Fe(NO₃)₃, FeSO₄, acide folique, glucose, H₂SeO₃, HEPES, hypoxanthine, insuline humaine, KC1, acide linoléique, acide lipoïque, MgCl₂, MnCl₂, MnSO₄, myo-inositol, Na₂HPO₄, Na₂SeO₃, Na₂SiO₃, NaCl, NaH₂PO₄, NaHCO₃, pyruvate de sodium, acétate de sodium, NH₄VO₃, NiCl₂, nicotinamide, rouge de phénol, polysorbate 80, putrescine, putrescine 2HCl, pyridoxine HCl, pyridoxal HCl, riboflavine, SnCl₂, thiamine HCl, thymidine, vitamine B₁₂ et ZnSO₄,
pour soutenir le processus de régénération de la peau, fournir un environnement optimal à la peau, améliorer la structure cutanée, améliorer l'état global de la peau, améliorer l'apparence de la peau et/ou aider la peau à regagner son élasticité et l'impression de santé.

2. Méthode selon la revendication 1, où les acides aminés comprennent un ou plusieurs des suivants : L-alanine, L-arginine, L-asparagine, acide L-aspartique, L-cystéine, L-cystine, glycine, L-glutamine, acide L-glutamique, L-histidine, L-isoleucine, L-leucine, L-lysine, L-méthionine, L-phénylalanine, L-proline, L-sérine, L-thréonine, L-tryptophane, L-tyrosine, L-valine et leurs sels de chlorhydrate.

3. Méthode selon la revendication 1, où au moins l'une des substances (a) et (b) est d'origine marine ou d'origine synthétique.

4. Méthode selon la revendication 1, où le glycosylaminoglycane comprend au moins l'un des membres du groupe constitué par le 4-sulfate de chondroïtine et le 6-sulfate de chondroïtine.

5. Méthode selon la revendication 1, où un rapport massique des substances (a) et (c) est compris entre 35:1 et 3:1.

6. Méthode selon la revendication 1, où un rapport massique des substances (a) et (b) est compris entre 10:1 et 1,5:1.

7. Méthode selon la revendication 1, où un rapport massique des substances (b) et (c) est compris entre 10:1 et 1:1.

8. Méthode selon la revendication 1, où le milieu de culture de cellules cutanées comprend au moins l'un
des membres du groupe constitué par DMEM/HAM F12 (1:1) et MCDB 153.

9. Méthode selon la revendication 1, où la préparation comprend en outre un tampon citrate.

10. Méthode selon la revendication 1, où la préparation comprend en outre au moins l'un des membres du groupe constitué par les suivants : Q10, alpha-glucosylrutine, un alpha-hydroxyacide, orotate de Zn, carnitine, créatine et taurine.
